(19) Europäisches Patentamt
European Patent Office
Office européen des brevets

(11) Publication number: **0 063 004**
**A1**

(12) # EUROPEAN PATENT APPLICATION

(21) Application number: 82301714.0

(22) Date of filing: 01.04.82

(51) Int. Cl.³: C 07 C 101/28
C 07 C 103/76, A 61 K 31/165
A 61 K 31/215

(30) Priority: 09.04.81 GB 8111252

(43) Date of publication of application:
20.10.82 Bulletin 82/42

(84) Designated Contracting States:
BE CH DE FR GB IT LI NL SE

(71) Applicant: BEECHAM GROUP PLC
Beecham House Great West Road
Brentford Middlesex(GB)

(72) Inventor: Smith, David Glynn
36 Shrewsbury Road
Redhill Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Secondary amines, processes for their preparation, and pharmaceutical compositions containing them.**

(57) The compounds of formula (I);

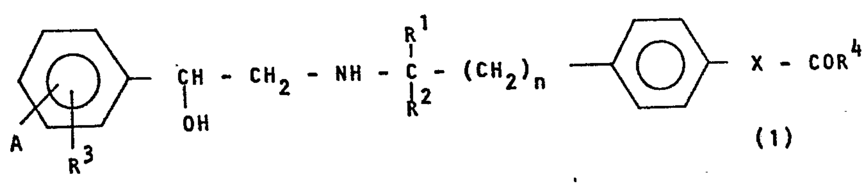

(1)

and salts thereof; wherein

$R^1$ is hydrogen or methyl;

$R^2$ is hydrogen or methyl;

$R^3$ is hydrogen, fluorine, chlorine, bromine, trifluoromethyl or $C_{1-6}$ alkyl;

n is 1 or 2;

X is $C_{1-6}$ straight or branched alkylene; and

$R^4$ is $C_{1-6}$ alkoxy, hydroxy or amino optionally substituted with one or more lower alkyl groups,

A is hydrogen, bromine, chlorine, or fluorine, have been found to possess anti-obesity and/or anti-hyperglycaemic activity.

EP 0 063 004 A1

SECONDARY AMINES, PROCESSES FOR THEIR PREPARATION, AND PHARMACEUTICAL COMPOSITIONS CONTAINING THEM.

The present invention relates to derivatives of ethanolamine which have anti-obesity and/or hypoglycaemic activity, to processes for their production and to their use in medicine.

European Patent Application No. 79301197.4 (Beechams) discloses compounds of formula

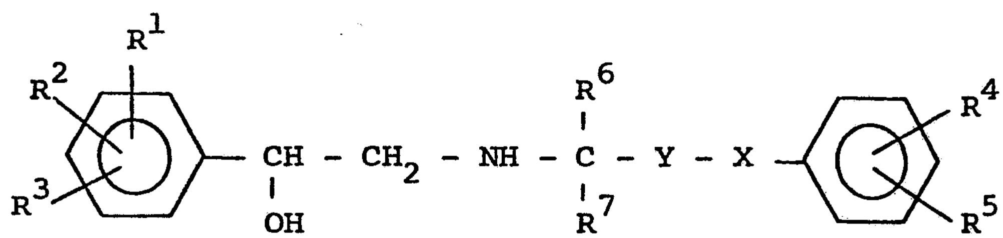

wherein $R^1$ is hydrogen, fluorine, chlorine, hydroxyl, hydroxymethyl, methyl, methoxy, amino, formamido, acetamido, methylsulphonamido, nitro, benzyloxy, methylsulphonylmethyl, ureido, trifluoromethyl or p-methoxybenzylamino;

$R^2$ is hydrogen, fluorine, chlorine or hydroxyl;

$R^3$ is hydrogen, fluorine, chlorine or hydroxyl;

$R^4$ is a carboxylic acid group or a salt, ester or amide thereof;

$R^5$ is hydrogen, fluorine, chlorine, methyl, methoxy, hydroxyl, or a carboxylic acid group or a salt, ester or amide thereof;

$R^6$ is hydrogen, methyl, ethyl or propyl;
$R^7$ is hydrogen, methyl, ethyl or propyl;
X is oxygen or a bond; and
Y is $C_{1-6}$ alkylene or a bond,

which possess anti-obesity and/or hypoglycaemic activity.

It has now been discovered that a class of novel arylethanolamine derivatives have anti-obesity and/or hypoglycaemic activity.

Accordingly, the present invention provides a compound of formula (I):

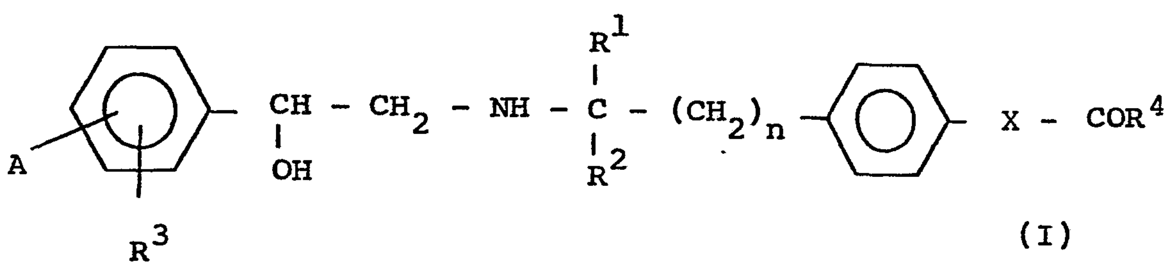

(I)

and salts thereof;

wherein $R^1$ is hydrogen or methyl;
$R^2$ is hydrogen or methyl;
$R^3$ is hydrogen, fluorine, chlorine, bromine, trifluoromethyl or $C_{1-6}$ alkyl;
n is 1 or 2;
X is $C_{1-6}$ straight or branched alkylene;
$R^4$ is $C_{1-6}$ alkoxy, hydroxy or amino optionally substituted with one or more lower alkyl groups; and
A is hydrogen, fluorine, chlorine or bromine.

Preferably n is 1.

Preferably $R^1$ is hydrogen and $R^2$ is methyl. X is suitably methylene, or ethylene. The $R^3$ group may be any position on the ring, but is preferably in the meta-position.

Pharmaceutically acceptable salts of compounds of formula (I) include acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicylic acid or acetylsalicyclic acid; and, when $R^4$ includes a carboxyl group, salts of that group such as alkali metal salts, including sodium and potassium salts, alkaline earth metal salts, including calcium and magnesium salts, and ammonium salts.

The salts of compounds of formula (I) need not be pharmaceutically acceptable as they are also useful in the preparation of other compounds of formula (I) and in the separation of stereoisomers of compounds of formula (I) when the salt ion is also optically active.

Compounds of formula (I) have at least one asymmetric carbon atom, ie the carbon atom bearing the

hydroxyl and substituted phenyl groups, and, when $R^1$ and $R^2$ are different, the carbon atom bearing $R^1$ and $R^2$ is also a centre of asymmetry. The compounds may, therefore, exist in at least two and often four stereoisomeric forms. The present invention encompasses all stereoisomers of the compounds of formula (I) whether free from other stereoisomers or admixed with other stereoisomers in any proportion and thus includes, for instance, racemic mixtures of optical isomers.

Preferably, the carbon atom bearing the hydroxyl and substituted phenyl groups has the R-configuration.

The most potent compounds of formula (I) are those wherein $R^1$ and $R^2$ are different and both asymmetric carbon atoms are in the R-configuration.

The absolute configuration of any compound of formula (I) may be determined by conventional X-ray crystallographic techniques.

It is believed that, in the $^{13}C$ nmr spectrum of compounds of formula (I) wherein one of $R^1$ and $R^2$ is hydrogen and the other is methyl, the diastereoisomer having the greater anti-obesity and anti-hyperglycaemic activity is that for which the signal of the methyl group carbon atom appears at higher field (the lower numerical value when expressed in ppm) in $D_6$-dimethyl sulphoxide solution. The paired resonances often appear at approximately 20 ppm (less active) and slightly below 20 ppm (more active) down field from tetramethylsilane. Other paired resonances can occur for the carbon atoms attached directly to the nitrogen atom and the carbon which carries the hydroxyl and phenyl groups. Again the paired resonances of the more active diastereoisomer of the investigated compounds appear at the higher field position.

The present invention provides a process for producing a compound of formula (I), or a salt thereof, which process comprises reducing an oxo-group and/or double or triple bond and/or cleaving and N-benzyl group of a compound of formula (II):

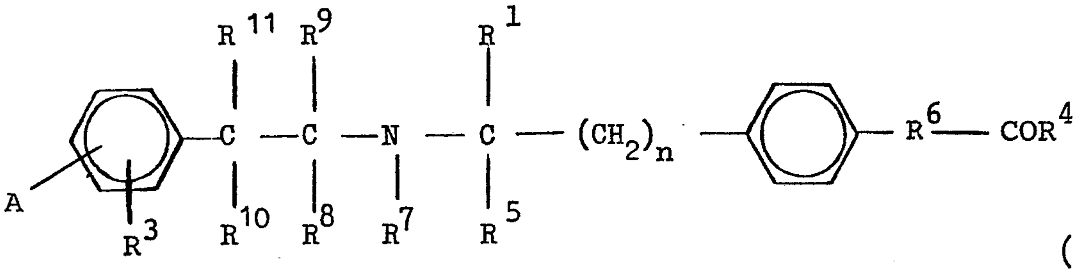

(II)

wherein $R^1$, $R^3$, $R^4$, A and n are as defined in relation to formula (I),

$R^5$ is a group $R^2$ as defined in relation to formula (I) or together with $R^7$ forms a bond,

$R^6$ is $C_{1-6}$ straight or branched chain alkylene, optionally containing a carbon-carbon double or triple bond,

$R^7$ is hydrogen or benzyl or together with $R^5$ or $R^8$ forms a bond,

$R^8$ is hydrogen or together with $R^9$ forms an oxo-group or together with $R^7$ forms a bond,

$R^9$ is hydrogen or together with $R^8$ forms an oxo-group,

$R^{10}$ is hydroxyl or together with $R^{11}$ forms an oxo-group,

$R^{11}$ is hydrogen or together with $R^{10}$ forms an oxo-group

provided that there is no more than one oxo-group represented by any of $R^8$ to $R^{11}$;

and optionally thereafter forming a salt of the compound of formula (I) so formed and/or converting the compound of formula (I) so formed into a further compound of formula (I).

Conversion of a compound of formula (I) into a further compound of formula (I) may be effected by, for instance, forming an amide from an ester, using conventional methods.

Where there are two or more reducible moieties in the compound of formula (II) these may be reduced separately in any order or simultaneously.

The aforementioned reductions may be effected by conventional chemical methods or by catalytic methods. Suitably, chemical reduction may be effected with, aluminium amalgam, lithium aluminium hydride, sodium cyanoborohydride or sodium borohydride. Catalytic hydrogenation may be carried out using catalysts such as palladium on charcoal, or platinum, for instance, as platinum oxide.

Reduction by sodium borohydride is conveniently effected in a lower alkanolic solvent such as methanol or ethanol. The reaction is generally carried out at from 0-20°C.

Reduction by lithium aluminium hydride is conveniently effected in a dry, ether solvent such as diethyl ether or tetrahydrofuran at ambient or elevated temperatures.

Catalytic reduction is conveniently effected in a conventional hydrogenation solvent such as a lower alkanol, for instance ethanol. The hydrogenation is generally carried out under hydrogen gas at about 1 to 10 atmospheres pressure and at ambient or elevated temperatures.

Reduction of a compound of formula (II) wherein $R^7$ is benzyl is conveniently effected by catalytic hydrogenation, preferably using palladium on charcoal as catalyst.

Preferred aspects of the process comprise reducing a compound of formula (IIA):

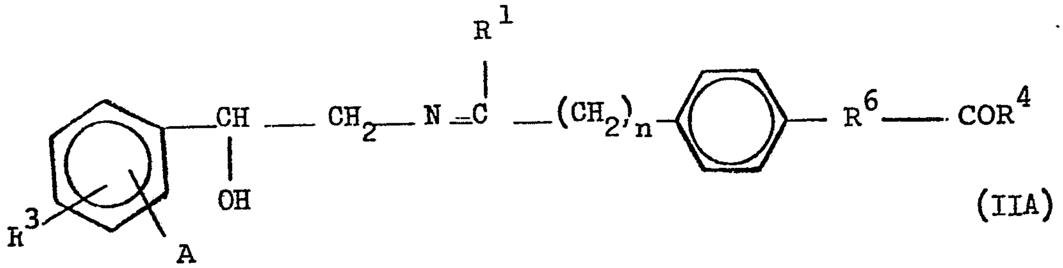

(IIA)

or reducing a compound of formula (IIB):

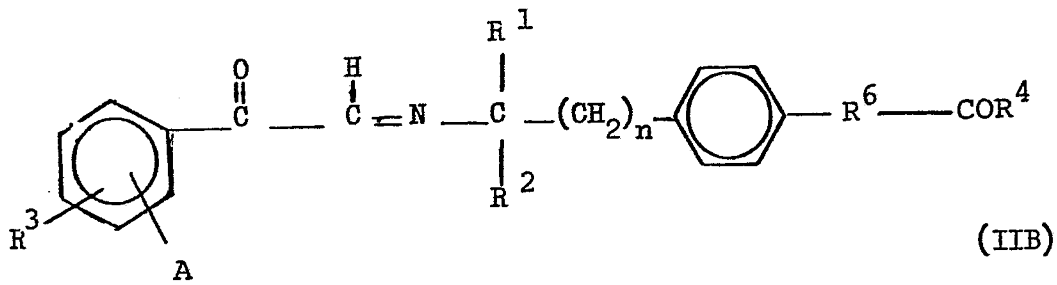

(IIB)

or reducing a compound of formula (IIC):

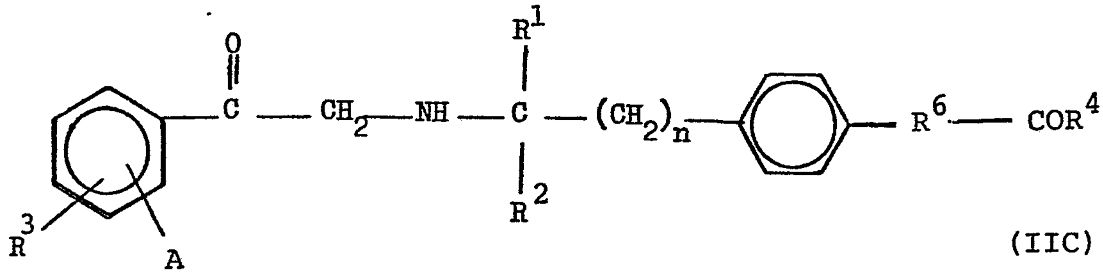

(IIC)

or reducing a compound of formula (IID)

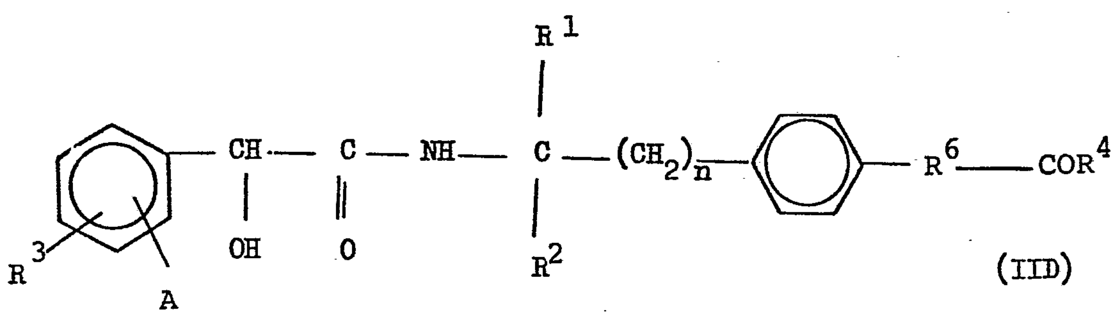

(IID)

or reducing a compound of formula (IIE):

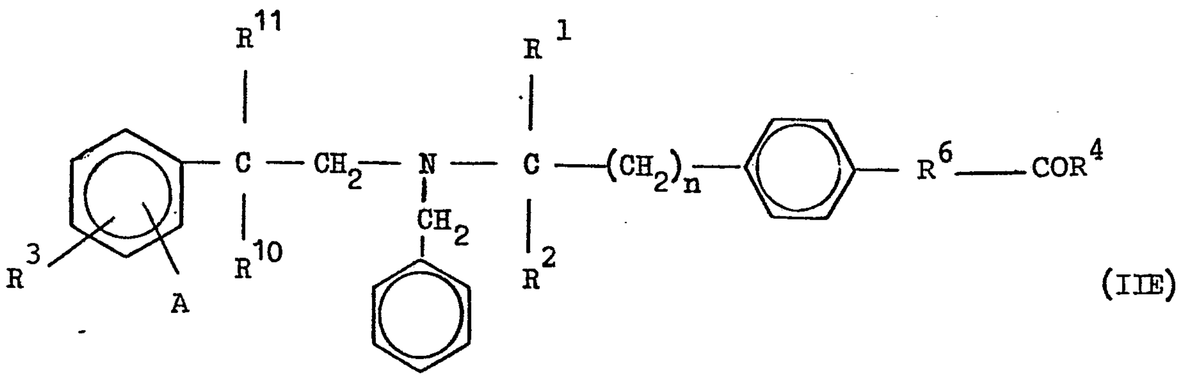

(IIE)

or reducing a compound of formula (II F):

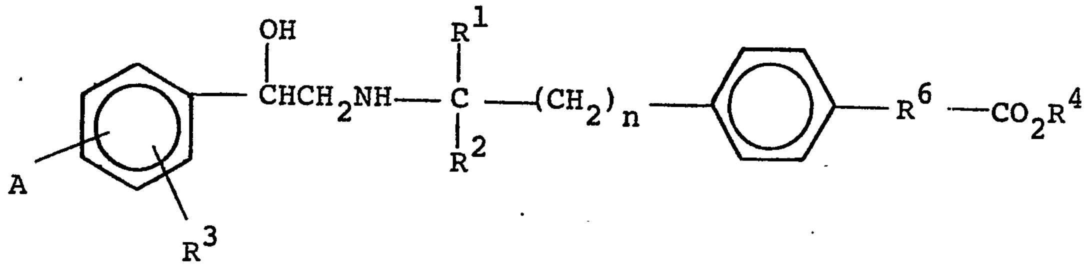

(II F)

wherein $R^1$, $R^2$, $R^3$, $R^4$, A and n are as defined in relation to formula (I) and $R^6$, $R^{10}$ and $R^{11}$ are as defined in relation to formula (II).

The present invention also provides another process for producing a compound of formula (I) or a salt thereof, which process comprises reacting a compound of formula (III):

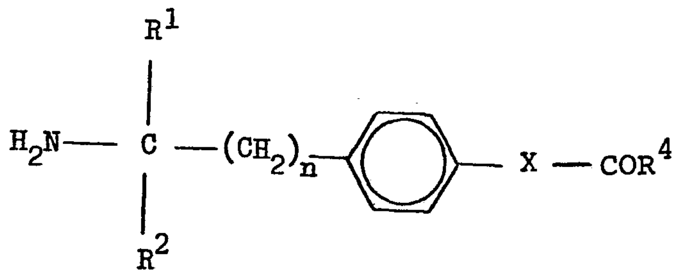

(III)

wherein $R^1$, $R^2$, $R^4$ X, and n are as defined in relation to formula (I);

with a compound of formula (IV):

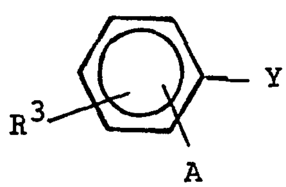

(IV)

wherein $R^3$ and A are as defined in relation to formula (I), and Y is a group capable of reacting with the amine of formula (III) thus forming a compound of formula (I), and optionally thereafter forming a salt of the compound of formula (I) so formed.

Typical examples of compounds of formula (IV) are compounds of formulae (IVA) and (IVB):

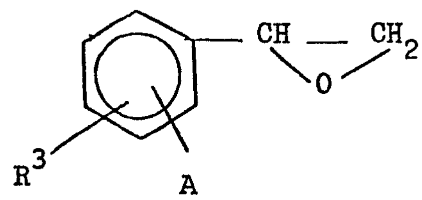

(IVA)

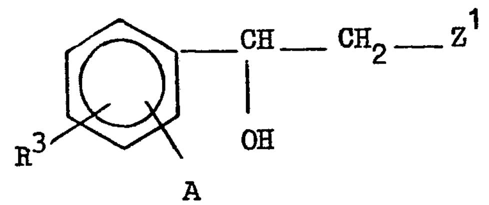

(IVB)

wherein $R^3$ and A are as defined in relation to formula (I) and $Z^1$ is a leaving group, preferably halogen or tosyloxy.

The reaction of a compound of formula (III) with a compound of formula (IVA) is conveniently effected in a solvent such as a lower alkanol, preferably ethanol.

The reaction of a compound of formula (III) with a compound of formula (IVB) is conveniently conducted in a solvent, such as dimethyl sulphoxide, at elevated temperature, preferably about 50°C, for about 3 days.

The present invention provides a further process for the production of compounds of formula (I) or salts thereof, which process comprises reacting a compound of formula (V):

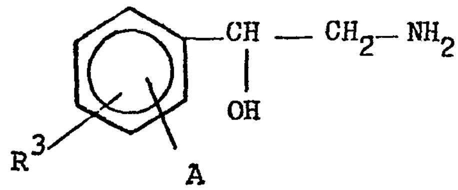

(V)

wherein $R^3$ and A are as defined in relation to formula (I);

with a compound of formula (VI):

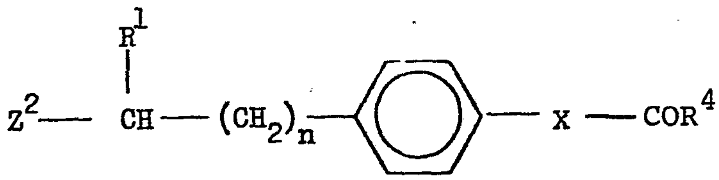

(VI)

wherein $R^1$, $R^4$, X and n are as defined in relation to formula (I), and $Z^2$ is a leaving group preferably halogen or tosyloxy.

The preparation of compounds of formula (V) is described in Published European Patent Application No. 0 021 636.

The reaction of a compound of formula (V) with a compound of formula (VI) is conveniently effected in a solvent such as dimethyl sulphoxide at an elevated temperature, preferably about 50°C for about two or three days.

A preferred process for producing compounds of formula (I) comprises the reduction of a compound of formula (IIA), especially using sodium borohydride in methanol at ambient temperatures.

The salts of compounds of formula (I) may be produced by treating the compound of formula (I) with the appropriate acid, or when the compound is a carboxylic acid by treatment with an appropriate base.

Compounds of formula (I) and salts thereof, produced by the above processes, may be recovered by conventional methods.

Compounds of formula (II) may be produced by reacting a compound of formula (IIIA):

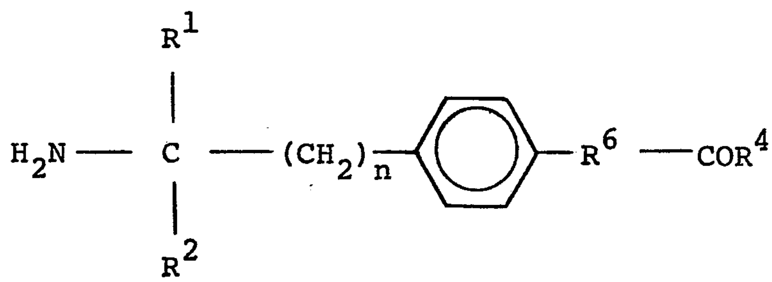

(IIIA)

wherein $R^1$, $R^2$, $R^4$ and n are as defined in relation to formula (I) and $R^6$ is as defined in relation to formula (II),

with a compound of formula (VII):

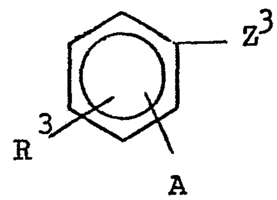

(VII)

wherein $R^3$ and A are as defined in relation to formula (I) and $Z^3$ is a group which is capable of reacting with the amine of formula (IIIA) thus forming a compound of formula (II). Typical examples of compounds of formula (VII) are:

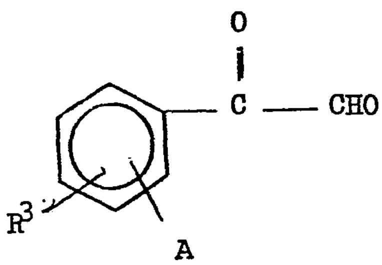

(VIIA)

or its hydrate or hemi-acetal of a lower alkanol;

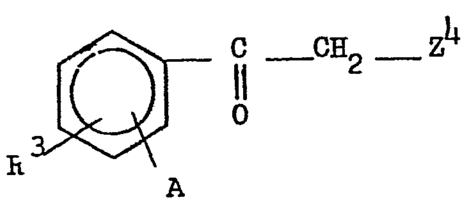

(VIIB)

wherein $Z^4$ is a leaving group, preferably bromine;

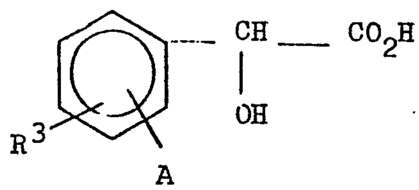

(VIIC)

wherein $R^3$ and A are as defined in relation to formula (I).

Conventional conditions suitable for use with the particular compound of formula (VII) may be used for this reaction. Thus the reaction of a compound of formula (VIIA) with a compound of formula (IIIA) is conveniently conducted at elevated temperatures under conditions resulting in the removal of the water formed during the reaction. A particularly suitable method is to perform the reaction in a solvent, such as benzene, heated under reflux and to remove the water azeotropically using a Dean and Stark trap.

The reaction of a compound of formula (VIIB) with a compound of formula (IIIA) is conveniently conducted in a polar organic solvent such as acetonitrile or butanone, at an elevated temperature, for instance heated under reflux.

The reaction of a compound of formula (VIIC) with a compound of formula (IIIA) is conveniently conducted under standard peptide formation reaction conditions.

Alternatively, a compound of formula (II) may be prepared by reacting a compound of formula (VIII):

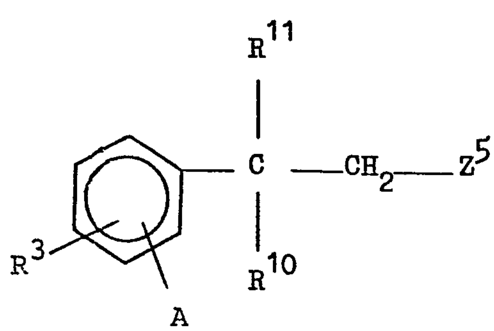

(VIII)

wherein $R^3$ and A are as defined in relation to formula (II);

and $Z^5$ is a leaving group, preferably halogen or tosyloxy, and $R^{10}$ and $R^{11}$ are as defined in relation to formula (II);

with a compound of formula (IX):

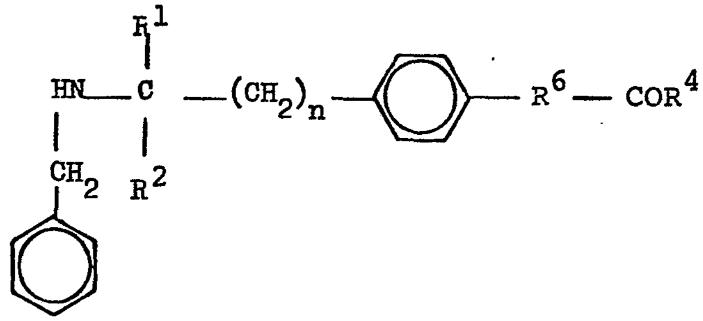

(IX)

wherein $R^1$, $R^2$, $R^4$, and n are as defined in relation to formula (I), and $R^6$ is as defined in relation to formula (II).

A particularly preferred process for producing certain compounds of formula (II) is by reacting a compound of formula (V) as hereinbefore defined with a compound of the formula (X):

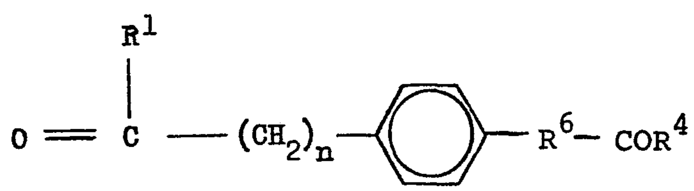

(X)

wherein $R^1$, $R^4$ and n are as defined in relation to formula (I), and $R^6$ is as defined in relation to formula (II).

The reaction of a compound of formula (V) with a compound of formula (X) is conveniently effected under conditions which result in the removal of water formed during the reaction. A particularly suitable method is to perform the reaction in a solvent, such as benzene, heated under reflux, and to remove the water azeotropically using a Dean and Stark trap.

It is often convenient to prepare the compound of formula (II) and reduce it, in situ, to the desired compound of formula (I) without isolation of the compound of formula (II).

Compounds of formula (I) having a single asymmetric carbon atom may, if desired, be resolved into individual enantiomers by conventional means, for example, by the use of an optically active acid as a resolving agent. Those compounds of formula (I) having two asymmetric carbon atoms may be separated into diastereoisomeric pairs of enantiomers by, for example, fractional crystallisation from a suitable solvent such as ethyl acetate. The pair of enantiomers thus obtained may be resolved into individual stereoisomers by conventional means such as by the use of an optically active acid as a resolving agent.

Suitable optically active acids which may be used as resolving agents are described in "Topics in Stereochemistry", Vol. 6, Wiley Interscience, 1971, Allinger, N.L., and Eliel, W.L. Eds.

Alternatively any enantiomer of a compound of formula (I) may be obtained by stereospecific synthesis using optically pure starting materials of known configuration.

By using single enantiomers of a compound of formula (IIIA) and of a compound of formula (VIIC) a stereospecific synthesis of a compound of formula (II) is achieved. This may then be reduced to a compound of formula (I) without altering the configuration of the two asymmetric carbon atoms. Thus, for example, reaction of a compound of formula (IIIA) with the R-absolute configuration and a compound of formula (VIIC) with the R-absolute configuration would afford

a compound of formula (II) and, on reduction, a compound of formula (I) with the R,R-absolute configuration.

By reacting a single enantiomer of a compound of formula (III) with a single enantiomer of a compound of formula (IVA) or (IVB), the direct stereospecific synthesis of a single enantiomer of a compound of formula (I) is effected. Thus, for example, reaction of a compound of formula (III) with the R-absolute configuration with a compound of formula (IVA) with the R-absolute configuration would afford a compound of formula (I) with the R,R-absolute configuration.

A compound of formula (I) or a pharmaceutically acceptable salt thereof (hereinafter "the drug") may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (I) or a pharmaceutically acceptable salt thereof with a pharmaceutically acceptable carrier therefor.

As used herein the terms "pharmaceutical composition" and "pharmaceutically acceptable" embrace compositions and ingredients for both human and veterinary use.

Usually the compositions of the present invention will be adapted for oral administration although compositions for administration by other routes, such as by injection are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or the like.

Typical carriers may, therefore, comprise such agents as microcrystalline cellulose, starch, sodium starch glycollate, polyvinylpyrrolidone, polyvinyl-polypyrrolidone, magnesium stearate, sodium lauryl sulphate, sucrose and the like.

Most suitably the composition will be provided in unit dose form. Such unit doses will normally comprise 0.1 to 500 mg of the drug, more usually 0.1 to 250 mg and favourably 0.1 to 100 mg.

The present invention further provides a method for treating obesity and/or hyperglycaemia in humans or domestic mammals, which method comprises administering an effective, non-toxic amount of a compound of formula (I) or a pharmaceutically acceptable salt thereof to obese and/or hyperglycaemic humans or domestic mammals.

In treating hyperglycaemic or obese humans the drug may be taken in doses, such as those described above, one to six times a day in a manner such that the total daily dose for a 70 kg adult will generally be about 0.1 to 1000 mg, and more usually about 1 to 500 mg.

In treating hyperglycaemic or obese animals, especially dogs, the drug may be administered by mouth, usually once or twice a day and at about 0.025 mg/kg to 10 mg/kg, for example 0.1 mg/kg to 2 mg/kg.

The invention will now be illustrated with reference to the following Examples, which are not intended to limit the scope in any way.

As used in the Examples, the term "mixture of diastereoisomers" refers to a mixture of two pairs of racemic stereoisomers having a diastereoisomeric relationship with each other.

EXAMPLE 1

N-(2-(4-(2-Carbomethoxyethyl)phenyl)-1-methylethyl)-2-hydroxy-2-(3-chlorophenyl) ethanamine

A mixture of 2-hydroxy-2-(3-chlorophenyl)ethanamine (1.72g) and 4-(E)-(2-carbomethoxyethenyl)phenylpropan-2-one (2.18g) was heated under reflux in benzene for 4 h using a Dean & Stark apparatus. The solvent was changed to ethanol and the reaction mixture hydrogenated over platinum oxide. Uptake of one mole of hydrogen proceeded rapidly after which time the catalyst was filtered, the solvent evaporated and the residue dissolved in tetrahydrofuran. This was added to aluminium amalgam in tetrahydrofuran and the reaction left at ambient temperature for 2h. The reaction mixture was filtered through celite, the solvent evaporated and the residue was crystallized and recrystallized from hexane to give the title compound (0.6g) as a 60:40 mixture of diastereoisomers, m.p. 68-70°C.

'Hnmr $\tau$ ($CDCl_3$) 8.95 (3H,d,J=6Hz), 7.0-7.6 (11H,m), 6.4 (3H,s), 5.5 (1H,m), 2.9 (4H,s), 2.7-2.9 (4H,m).

EXAMPLE 2

N-(2-(4-(2-Carbomethoxyethyl)phenyl)-1-methylethyl)-2-hydroxy-2-(3-trifluoromethylphenyl) ethanamine

N-(2-(4-((E)-2-Carbomethoxyethenyl)phenyl)-1-methylethyl)-2-hydroxy-2-(3-trifluoromethylphenyl)ethanamine, (1.0g, consisting of a >75:<25 mixture of diastereoisomers) was hydrogenated over 10% Pd/C in ethanol. The mixture was filtered, the ethanol evaporated and the residue was chromatographed on silica gel. Elution with 2% methanol-chloroform gave the title compound isolated as an 88:12 mixture of diastereoisomers (0.2g) m.p. 61-70°C.

'Hnmr, τ($CDCl_3$) 8.9 (3H,d,J=6Hz), 6.9-7.7 (11H,m), 6.4 (3H,s), 5.75 (1H,m), 2.9 (4H,s), 2.4-2.7 (4H,m).

## EXAMPLE 3

### N-(2-(4-(Carbomethoxymethyl)phenyl)-1-methylethyl)-2-hydroxy-2-(3-chlorophenyl)ethanamine

(4-Carbomethoxymethyl)phenylpropanone (0.8g) and 2-hydroxy-2-(3-chlorophenyl)ethanamine (0.7g) were heated under reflux in benzene using a Dean & Stark head. The solvent was evaporated, methanol added followed by the portionwise addition of sodium borohydride. The solvent was evaporated, the residue partitioned between ether and water and the ether layers dried, (magnesium sulphate). Evaporation of the solvent gave an oil which was chromatographed on silica gel. Elution with 1% methanol-chloroform gave the title compound isolated as the fumarate salt.

'Hnmr,τ($d_6$ DMSO) 8.95 (3H,s), 6.7-7.4 (5H,m), 6.45 (3H,s), 6.4 (2H,s), 5.1 (1H,m), 3.5 (1H,s), 3.0-3.5 (3H, broad), 2.4-3.1 (8H,m).

## EXAMPLE 4

### N-(2-(4-(2-N'-Methylcarboxamidoethyl)phenyl)-1-methylethyl)-2-hydroxy-2-(3-trifluoromethylphenyl)ethanamine

A mixture of N-(2-(4- E -(2-carbomethoxyethenyl)-phenyl)-1-methylethyl)-2-hydroxy-2-(3-trifluoromethylphenyl) ethanamine (4.3g) and aluminium amalgam (from aluminium foil (2.8g) and mercuric chloride (1.4g)) in tetrahydrofuran was left at ambient temperature for 2h. The reaction mixture was filtered and the solvent evaporated to give the corresponding propionate which, without further purification, was heated at 100$^{o}$ for 5h in an autoclave with ethanolic methylamine. The solution was evaporated and the residue chromatographed on silica gel. Elution with 8% methanol-chloroform gave the title amide mp 122-127$^{o}$C (benzene).

'Hnmr, τ($d_6$DMSO) 9.1 (3H,d,J=6Hz), 6.4-7.8 (10H(m) + 3H, d,J=6 $H_z$, collapses to a singlet with $D_2O$ + 2H, disappears with $D_2O$), 5.3 (1H,m), 2.9 (4H,s), 2.1-2.5 (4H,m).

EXAMPLE 5

N-(2-(4-(1-Carbomethoxy-1-methylethyl)phenyl)-1-methylethyl)-2-hydroxy-2-(3-trifluoromethylphenyl)ethanamine hydrogen bromide.

2-Hydroxy-2-(3-trifluoromethylphenyl)ethanamine (3.6g) and 4-(1-carbomethoxy-1-methylethyl)phenylpropan-2-one (4.0g) were heated, for 4 hours, in benzene under reflux with the removal of water. The solvent was evaporated and the residue taken up in methanol and treated at 0°C with sodium borohydride (4.0g). After evaporation, dilution with water and extraction into ethyl acetate, the crude product was chromatographed on silica eluting with 3% methanolic chloroform to give an oil (5.5g). This was treated with ethereal hydrogen bromide to give the title compound, mp 110-111.5°C, (ether-cyclohexane), as a 51:49 mixture of diastereoisomers.

'Hnmr, τ($d_6$DMSO) 8.7-8.9 (3H,d), 8.45 (6H,s), 7.1-7.4 (1H, m), 6.3-7.0 (7H,m+s), 4.8 (1H,m), 3.7 (1H, broad, disappears with $D_2O$), 2.7 (4H,s), 2.1-2.4 (4H,m), 1.1 (2H, broad, disappears with $D_2O$).

EXAMPLE 6

N-(2-(4-(2-Carbomethoxyethyl)phenyl)-1-methylethyl)-2-hydroxy-2-(3-fluorophenyl)ethanamine

This was prepared in an identical manner to the compound described in Example 2 using N-(2-(4-( E -2-carbomethoxyethenyl)phenyl)-1-methylethyl)-2-hydroxy-2-(3-fluorophenyl)ethanamine. The title compound was isolated as the hydrochloride salt, mp 116-130°C (ethyl acetate-ether) as a 87:13 mixture of diastereoisomers.

'Hnmr, $\tau$($d_6$ DMSO) 8.85 (3H,d,J=6Hz), 7.0-7.5 (6H,m), 6.4-7.0 (4H,m), 6.4 (3H,s), 4.8 (1H,m), 3.55 (1H, broad, disappears with $D_2O$), 2.35-3.0 (8H,m), 0.0-1.0 (2H, broad, disappears with $D_2O$).

EXAMPLE 7

N-(2-(4-(2-Carbomethoxyethyl)phenyl)-1-methylethyl)-2-hydroxy-2-(2-fluorophenyl)ethanamine

This was prepared in an identical manner to the compound described in Example 2 using N-(2-(4-( E -2-carbomethoxyethenyl)phenyl)-1-methylethyl)-2-hydroxy-2-(2-fluorophenyl)ethanamine. The title compound was isolated as the hydrochloride salt, mp 120-131 °C (ethyl acetate) as a 40:60 mixture of diastereoisomers.

'Hnmr, τ($d_6$ DMSO) 8.85 (3H,d,J=6Hz), 7.0-7.6 (6H,m), 6.4-7.0 (4H,m), 6.4 (3H,s), 4.6 (1H,m), 3.6 (1H, broad, disappears with $D_2O$). 2.8 (4H,s), 2.2-2.9 (4H,m), 0.0-1.0 (2H, broad, disappears with $D_2O$).

EXAMPLE 8

N-(2-(4-(2-Carbomethoxy-1-methylethyl)phenyl)ethyl)-2-hydroxy-2-phenylethanamine

This was prepared in an identical manner to the compound described in Example 2 using N-(2-(4-(2-carbomethoxy-1-methylethenyl)phenyl)ethyl)-2-hydroxy-2-phenylethanamine. The title compound was isolated as the hydrochloride salt, mp 117-127 $^{o}$C (ethylacetate).

'Hnmr, $\tau$($d_6$ DMSO) 8.8 (3H,d,J=8Hz), 7.45 (3H,d,J=6Hz), 6.6-7.1 (9H,m), 6.45 (3H,s), 4.85 (1H,m), 3.7 (1H, broad, disappears with $D_2O$) 2.75 (4H,s), 2.4-3.0 (5H,m), 0.45 (2H, broad disappears with $D_2O$).

EXAMPLE 9

N-(3-(4-(2-Carbomethoxy-1-methylethyl)phenyl)-1,1-dimethylpropyl)-2-hydroxy-2-phenylethanamine

This was prepared in an identical manner to the compound described in Example 2 using N-(3-(4-(2-carbomethoxy-1-methylethenyl)phenyl)-1,1-dimethylpropyl)-2-hydroxy-2-phenylethanamine. The title compound was isolated as the hydrochloride salt, mp 88-94°C.

'Hnmr, $\tau$($d_6$ DMSO) 8.8 (3H,d,J=8Hz), 8.65 (6H,s), 8.05 (2H,m), 7.45 (3H,d,J=6Hz + 2H,m), 6.9 (4H,m), 6.65 (1H, m), 6.5 (3H,s), 4.9 (1H,m), 3.75 (1H, disappears with $D_2O$), 2.8 (4H,s), 2.6 (5H,m), 1.75 (1H, broad, disappears with $D_2O$), 0.25 (1H, broad, disappears with $D_2O$).

DESCRIPTION 1

(4-Carbomethoxymethyl)phenyl propanone

Potassium cyanide (1.6g) was added to a solution of (4-bromomethyl)phenylpropanone, protected as the ethylene ketal (5.9)g), in dimethyl sulphoxide and the mixture heated at $60^{o}$ until tlc showed the disappearance of starting material. The mixture was diluted with water, extracted with ether and the ethereal layers washed with water (x3), dried and evaporated to give (4-cyanomethyl) phenylpropanone ethylene ketal (2.0g). The nitrile was dissolved in 50% sulphuric acid (50ml) and heated for 16h under reflux. When cool the product was extracted into ether and the extracts dried and evaporated. The residue (1g) was taken up in methanol (50ml), conc. sulphuric acid (0.5ml) added and the mixture was heated under reflux for 5h. When cool sodium bicarbonate solution was added until neutral, the methanol evaporated and the product extracted into ether. The ether layers were dried and evaporated to give the title compound (0.8g).

'Hnmr, $\tau$($CDCl_3$) 7.85 (3H,s), 6.45 (2H,s), 6.35 (3H,s+2H,s), 2.7-3.1 (4H,m).

DESCRIPTION 2

4-(1-Carbomethoxy-1-methylethyl)phenylpropan-2-one

4-(1-Carbomethoxy-1-methylethyl)benzaldehyde (20.3g) was heated with n-butylamine (13.5ml) in benzene under reflux with removal of water. The solvent was evaporated and the residue heated at 100-110$^{o}$C for 1 hour with nitroethane (17.5ml) in glacial acetic acid (150ml). The solution was cooled, diluted with water and extracted with dichloromethane. The organic layers were combined, washed with sodium bicarbonate solution and dried. Evaporation of the solvent gave 1-(4-(1-carbomethoxy-1-methylethyl) phenyl-2-nitroprop-1-ene as an oil (25.9g).

'Hnmr, $\tau$($CDCl_3$) 8.35 (6H,s), 7.5 (3H,s), 6.3 (3H,s), 2.5-2.9 (4H,m), 1.95 (1H,s).

The above nitropropene (25.9g) was added to aluminium amalgam, (from aluminium (17.3g) and mercuric chloride (8.6g))in tetrahydrofuran. The mixture was filtered and the filtrate evaporated. The residue was partitioned between water and dichloromethane and the organic layer dried and evaporated to give the above propanone oxime (17.8g). This was heated with sodium metabisulphite (62.3g) for 18 hours in methanol (300ml) and water (150ml). The reaction mixture was cooled and treated with conc. sulphuric acid (40ml), then extracted with dichloromethane to give the title compound as an oil (13.5g) bp 142-145$^{o}$C/ 0.7mm.

'Hnmr, $\tau$($CDCl_3$) 8.4 (6H,s), 7.85 (3H,s), 6.3 (2H,s + 3H,s), 2.6-2.95 (4H,m).

## DEMONSTRATION OF EFFECTIVENESS OF COMPOUNDS

### (i) ANTI-OBESITY ACTIVITY

The compound was administered by oral gavage in water or carboxymethyl-cellulose suspension to genetically obese mice daily for 28 days. At the end of the time the carcass composition was determined. The result obtained was as follows:-

| COMPOUND OF EXAMPLE NO. | DOSE mg/kg p.o. | g LIPID/MOUSE TREATED | CONTROL |
|---|---|---|---|
| 4 | 11.4 | 17.47 | 21.68 |

DEMONSTRATION OF EFFECTIVENESS OF COMPOUNDS

(ii) Effect on energy expenditure

The effect of the compounds on the energy expenditure of mice was demonstrated by means of the following procedure:

Female CFLP mice each weighing approximately 24 g, were given food and water *ad lib* before and during the experiment. The compounds were dissolved in water by addition of one mole of hydrochloric acid per mole of compound and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 hours after dosing from the volume of air leaving the boxes and its oxygen content, following the principles described by J.B. de V. Weir, *J. Physiol.* (London) 109, 1-9, (1949). The food intake of the mice was measured over this same period of 21 hours. The results are expressed as a percentage of the mean food intake or rate of energy expenditure of the mice dosed with water.

| COMPOUNDS OF EXAMPLE NO. | DOSE mg/kg p.o. | MEAN ENERGY EXPENDITURE | | MEAN FOOD INTAKE |
|---|---|---|---|---|
| | | (0-3h) | (0-21h) | |
| 1 | 21 | 167 | 133 | 85 |
| 2 | 22.8 | 153 | 125 | 85 |
| 3 | 23 | 166 | 127 | 89 |
| 4 | 19.6 | 158 | 114 | 81 |
| 5 | 28.1 | 126 | 96 | 102 |
| 6 | 22.0 | 154 | 132 | 65 |
| 7 | 22.0 | 139 | 122* | 107 |
| 8 | 21.1 | 108 | 99* | 88 |
| 9 | 23.3 | 142 | 106** | 85 |

* 0-15h, ** 0-19h

(iii) Cardiac activity

Rat hearts were perfused by the Langendorff procedure. Hearts were dissected free within 30 seconds of death and reverse perfused via the aorta and coronary vessels with Knebs-Ringer bicarbonate solution (pH 7.4, 37°C) gassed with 95% oxygen: 5% carbon dioxide at a flow rate between 8-12cm$^3$/minute. Responses were observed after injection of drug dissolved in isotonic saline into the perfusion media. Heart rate and tension were displayed on an Ormed MX2P recorder via a tension transducer and heart ratemeter.

Results are expressed as a percentage of the maximum response due to salbutamol.

| COMPOUND OF EXAMPLE NO: | DOSE ADDED TO PERFUSATE (μg) | HEART TENSION | HEART RATE |
|---|---|---|---|
| 1 | 30 | 40 | 60 |
| 2 | 10 | 12 | 10 |
| 3 | 30 | 44 | 100 |
| 4 | 30 | 20 | 10 |
| 5 | 30 | 0 | 0 |
| 6 | 30 | 108 | 0 |

(iv) Hypoglycaemic activity

Female CFLP mice, weighing approximately 25g, were fasted for 24 hours prior to the study. The compounds under study were administered orally as an aqueous solution to each of 6 mice. 30 minutes later a blood sample (20µl ) was obtained from the tail for the analysis of blood glucose. Immediately after taking this blood sample, glucose (1g/Kg body weight) was administered subsutaneously to each mouse. 6 mice were given water as a control. Blood samples were then obtained from each mouse at 30 minute intervals for 120 minutes.

Compounds that produced a significant ($P<0.05$) reduction of blood glucose, compared with control mice given water, at any time interval, were considered active. The area under the blood glucose curve over the 2 hour period after the administration of the glucose was calculated for each compound and compared with the value for control animals.

| COMPOUNDS OF EXAMPLE NO. | DOSE | % REDUCTION IN AREA UNDER BLOOD GLUCOSE CURVE |
|---|---|---|
| 3 | 1 mg $kg^{-1}$ | 58.6 |
| 8 | 50 µ mol $kg^{-1}$ | 54 |

CLAIMS

1. A compound of formula (I);

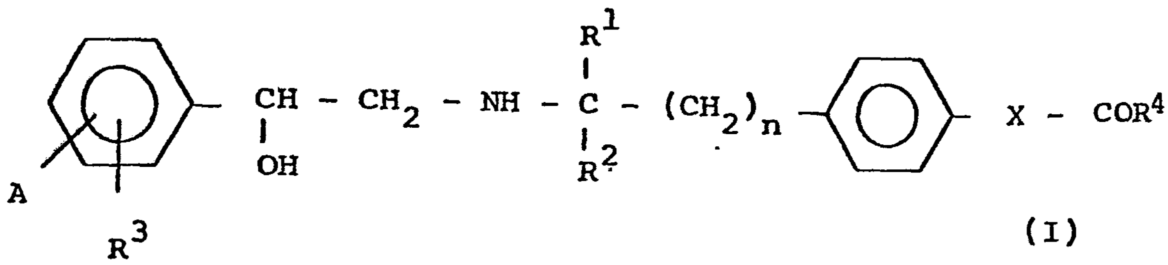

(I)

and salts thereof;

wherein $R^1$ is hydrogen or methyl;

$R^2$ is hydrogen or methyl;

$R^3$ is hydrogen, fluorine, chlorine, bromine, trifluoromethyl or $C_{1-6}$ alkyl;

n is 1 or 2;

X is $C_{1-6}$ straight or branched alkylene;

$R^4$ is $C_{1-6}$ alkoxy, hydroxy or amino optionally substituted with one or more lower alkyl groups; and

A is hydrogen, bromine, chlorine, or fluorine.

2. A compound according to claim 1, in which X is a methylene or ethylene group.

3. A compound according to claim 1 or 2 in which $R^3$ is in the meta-position.

4. A compound according to any one of claims 1 to 3, in which $R^1$ is hydrogen and $R^2$ is methyl.

5. A compound according to claim 1, selected from

N-(2-(4-(carbomethoxymethyl)phenyl)-1-methylethyl)-2-hydroxy-2-(3-chlorophenyl)ethanamine,

N-(2-(4-(2-N'-methylcarboxamidoethyl)phenyl)-1-methylethyl)-2-hydroxy-2-(3-trifluoromethylphenyl) ethanamine,

N-(2-(4-(1-carbomethoxy-1-methylethyl)phenyl)-1-methylethyl)-2-hydroxy-2-(3-trifluoromethylphenyl) ethanamine hydrogen bromide,

N-(2-(4-(2-carbomethoxyethyl)phenyl)-1-methylethyl)-2-hydroxy-2-(3-fluorophenyl)ethanamine,

N-(2-(4-(2-carbomethoxyethyl)phenyl)-1-methylethyl)-2-hydroxy-2-(2-fluorophenyl)ethanamine,

N-(2-(4-(2-carbomethoxy-1-methylethyl)phenyl)ethyl)-2-hydroxy-2-phenylethanamine,

N-(3-(4-(2-carbomethoxy-1-methylethyl) phenyl)-1,1-dimethylpropyl)-2-hydroxy-2-phenylethanamine,

N-(2-(4-(2-carbomethoxyethyl)phenyl)-1-methylethyl)-2-hydroxy-2-(3-chlorophenyl)ethanamine, and

N-(2-(4-(2-carbomethoxyethyl)phenyl)-1-methylethyl)-2-hydroxy-2-(3-trifluoromethylphenyl)ethanamine.

6. A process for producing a compound of formula (I) or a salt thereof, as defined in claim 1, which process comprises reducing an oxo-group, and/or a double or triple bond and/or cleaving an N-benzyl group of a compound of formula (II):

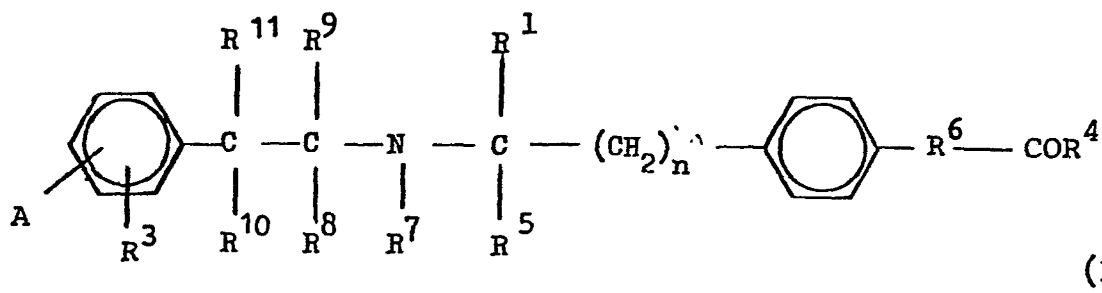

(II)

wherein $R^1$, $R^3$, $R^4$, A and n are as defined in relation to formula (I),

$R^5$ is a group $R^2$ as defined in relation to formula (I) or together with $R^7$ forms a bond,

$R^6$ is $C_{1-6}$ straight or branched chain alkylene, optionally containing a carbon-carbon double or triple bond,

$R^7$ is hydrogen or benzyl or together with $R^5$ or $R^8$ forms a bond,

$R^8$ is hydrogen or together with $R^9$ forms an oxo-group or together with $R^7$ forms a bond,

$R^9$ is hydrogen or together with $R^8$ forms an oxo-group,

$R^{10}$ is hydroxyl or together with $R^{11}$ forms an oxo-group,

$R^{11}$ is hydrogen or together with $R^{10}$ forms an oxo-group

provided that there is no more than one oxo-group represented by any of $R^8$ to $R^{11}$.

7. A process for preparing a compound of formula (I) or a salt thereof, as defined in claim 1, which process comprises reacting a compound of formula (III):

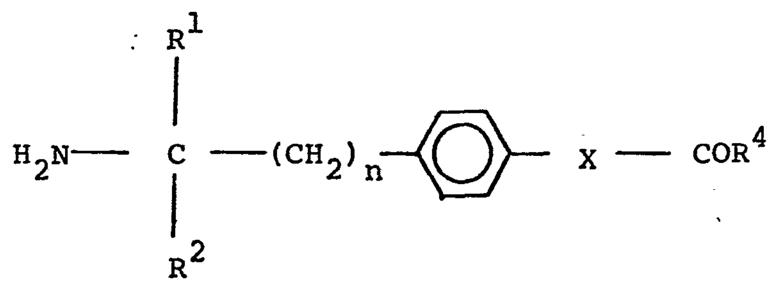

(III)

wherein $R^1$, $R^2$, $R^4$, X and n are as defined in relation to formula (I),

with a compound of formula (IV):

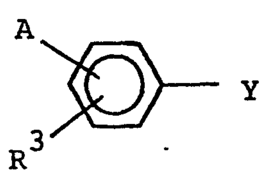

(IV)

wherein $R^3$ and A are as defined in relation to formula (I), and Y is a group capable of reacting with the amino group of formula (III),
and optionally thereafter forming a salt of the compound of formula (I) so formed.

8. A pharmaceutical composition comprising a compound of formula (I), as defined in any one of claims 1 to 5, or a pharmaceutically acceptable salt thereof, in combination with a pharmaceutically acceptable carrier therefor.

9. A compound of formula (I) as defined in any one of claims 1 to 5, or a pharmaceutically acceptable salt thereof, for use in the treatment of obesity or hyperglycaemia.

European Patent Office

# EUROPEAN SEARCH REPORT

0063004

Application number

EP 82 30 1714

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 006 735 (BEECHAM) *Claims* | 1,8-9 | C 07 C 101/28<br>C 07 C 103/76<br>A 61 K 31/165<br>A 61 K 31/215 |
| | --- | | |
| Y | EP-A-0 025 331 (BEECHAM) *Claims* | 1,8-9 | |
| | ----- | | |
| | | | TECHNICAL FIELDS SEARCHED (Int. Cl. 3) |
| | | | C 07 C 101/00<br>C 07 C 103/00 |

The present search report has been drawn up for all claims

| Place of search | Date of completion of the search | Examiner |
|---|---|---|
| THE HAGUE | 25-06-1982 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82